(19) **Europäisches Patentamt**
European Patent Office
Office européen des brevets

(11) **EP 1 975 247 A1**

(12) **EUROPÄISCHE PATENTANMELDUNG**

(43) Veröffentlichungstag:
**01.10.2008 Patentblatt 2008/40**

(51) Int Cl.:
*C12Q 1/68* (2006.01)   *G01N 21/64* (2006.01)
*G01N 33/543* (2006.01)

(21) Anmeldenummer: **07006650.1**

(22) Anmeldetag: **30.03.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder:
- **Micronas GmbH**
  **79108 Freiburg i. Br. (DE)**
- **Micronas Holding GmbH**
  **79108 Freiburg i.Br. (DE)**

(72) Erfinder:
- **Lehmann, Mirko, Dr. rer.nat.**
  **79117 Freiburg (DE)**
- **Freund, Ingo, Dipl.-Ing.**
  **79117 Freiburg (DE)**
- **Mory, Sonja**
  **79108 Freiburg (DE)**
- **Klapproth, Holger, Dr.**
  **79108 Freiburg (DE)**

(74) Vertreter: **Huwer, Andreas**
  **Grünwälderstrasse 10-14**
  **Postfach 1305**
  **79013 Freiburg i. Br. (DE)**

(54) **Biochip und Verfahren zu dessen Herstellung**

(57)   Bei einem Verfahren zur Herstellung eines Biochips (1) werden auf einem Träger (2) Rezeptoren (5) immobilisiert, die jeweils mindestens eine freie Bindungsstelle (6) haben, die für einen nachzuweisenden Liganden (9) bindungsspezifisch ist. An die Rezeptoren (5) werden außerhalb der freien Bindungsstellen (6) optische Marker (8) gebunden. In Abhängigkeit von der Bindung der einzelnen Marker (8) an die Rezeptoren (5) wird eine Lumineszenzstrohlung (11) erzeugt. Zur Überprüfung der Flächenbelegung des Trägers (2) mit den Rezeptoren (5) wird ein Messsignal für die Lumineszenzstrahlung (11) erfasst.

Fig. 2

**Beschreibung**

Die Erfindung betrifft ein Verfahren zur Herstellung eines Biochips, wobei auf einem Träger Rezeptoren immobilisiert werden, die jeweils mindestens eine freie Bindungsstelle haben, die für einen nachzuweisenden Liganden bindungsspezifisch ist. Ferner betrifft die Erfindung einen Blochip, mit einem Träger auf dem Rezeptoren immobilisiert sind, die jeweils mindestens eine freie Bindungsstelle haben, die für einen nachzuweisenden Liganden bindungsspezifisch ist.

Ein derartiges Verfahren, bei dem in einem Lösungsmittel gelöste Rezeptoren mittels eines Druckers an auf der Oberfläche eines Trägers befindliche Teststellen aufgebracht und dann auf dieser immobilisiert werden, ist aus der Praxis bekannt. Dabei sind die Teststellen matrixförmig in mehreren Reihen und Spalten angeordnet und durch Zwischenräume seitlich voneinander beabstandet. Beim Bedrucken der Träger kann es zu Fehlern kommen, beispielsweise wenn die Größe des von der Drucknadel auf den Träger übertragenen Tröpfchens von einem vorgegebenen Sollwert abweicht oder das Tröpfchen nicht an der vorgesehenen Stelle auf dem Träger positioniert und beispielsweise mit einer benachbarten Teststelle des Trägers in Kontakt gerät. Auch kann es vorkommen, dass eine flächige Teststelle nur bereichsweise mit Rezeptoren beschichtet wird, beispielsweise nur am Rand der Teststelle.

Um derartige Fehler möglichst zu vermeiden, werden die nach dem Verfahren hergestellten Biochips stichprobenartig überprüft. Bei der Überprüfung werden die Teststellen mit einem Analyten in Kontakt gebracht, der in einem Fluid Liganden enthält, die für die auf dem Träger immobilisierten Rezeptoren bindungsspezifisch sind. Die Liganden sind mit einem optischen Marker, also einem Molekül, das durch eine chemische Reaktion und/oder durch Anregung mit einer Anregungsstrahlung zur Aussendung von Lumineszenzstrahlung angeregt werden kann, markiert. Der Analyt wird derart mit den Rezeptoren in Kontakt gebracht, dass im Wesentlichen an jeden auf dem Träger befindlichen Rezeptor jeweils mindestens ein Liganden-Marker-Komplex bindet. Danach werden eventuelle noch vorhandene freie Liganden-Marker-Komplexe von der Oberfläche des Trägers entfernt. Dann werden die Marker der an die Rezeptoren gebundenen Liganden zur Aussendung von Lumineszenzstrahlung angeregt, um die Lumineszenzstrahlung ortsaufgelöst zu detektieren. Das Verfahren hat jedoch den Nachteil, dass die getesteten Biochips nicht mehr für eine weitere Messung verwendet werden können. Eine individuelle Überprüfung aller nach dem Verfahren hergestellter Biochips ist deshalb nicht möglich.

Aus DE 102 51 757 B4 Ist ferner ein Verfahren der eingangs genannten Art bekannt, bei dem die Rezeptoren auf einem Träger immobilisiert werden, in den Flächenbelegungssensoren integriert sind. Mit Hilfe der Flöchenbelegungssensoren wird die von der Anwesenheit der Rezeptoren abhängige elektrische Impedanz bzw. die Kapazität an der Oberfläche des Trägers gemessen. Für eine Überprüfung der Biochips auf Fertigungsfehler Ist das Verfahren jedoch nur beding geeignet, da die Flächenbelegungssensoren relativ teuer und aufwändig sind. AuBerdem kann das Messergebnis durch an der Oberfläche des Trägers befindliche Verunreinigungen, die ebenfalls eine Änderung der Impedanz bewirken können, verfälscht werden.

Es besteht deshalb die Aufgabe, ein Verfahren und einen Biochip der eingangs genannten Art zu schaffen, die auf einfache Weise eine individuelle Überprüfung der Flächenbelegung des Trägers mit den Rezeptoren ermöglichen.

Diese Aufgabe wird bezüglich des Verfahrens dadurch gelöst, dass zumindest einige Rezeptoren zusätzlich zu der mindestens einen ersten Bindungsstelle jeweils wenigstens eine zweite Bindungsstelle haben, die für den Liganden nicht bindungsspezifisch ist, dass an die zweiten Bindungsstellen optische Marker gebunden werden, dass in Abhängigkeit von der Bindung der einzelnen Marker an die Rezeptoren eine Lumineszenzstrahlung erzeugt wird, und dass zur Überprüfung der Flächenbelegung des Trägers mit den Rezeptoren ein Messsignal für die Lumineszenzstrahlung erfasst wird.

In vorteilhafter Weise ist es dadurch möglich, durch Messung der von den Markern erzeugten Lumineszenzstrahlung die Flächenbelegung des Trägers mit den Rezeptoren zu überprüfen, Da die Marker außerhalb der ersten Bindungsstellen an die Rezeptoren gebunden werden, kann der Biochip auch nach der Überprüfung der Flächenbelegung weiterhin zum Nachweisen und/oder zum Bestimmen der Konzentration des Liganden in einer zu untersuchenden Probe verwendet werden. Das Verfahren ermöglicht also eine individuelle Überprüfung der Flächenbelegung jedes einzelnen Biochlps. Bevorzugt werden alle auf dem Träger befindlichen Rezeptoren jeweils an einer zweiten Bindungsstelle mit dem Marker markiert. Es ist aber auch möglich, dass nur ein bestimmter Anteil der vorhandenen Rezeptoren optisch markiert wird. Dies kann beispielsweise dadurch erreicht werden, dass nur ein vorbestimmter, vorzugsweise gleichmäßig über den mit den Rezeptoren bedeckten Oberflächenbereich des Trägers verteilter Anteil der Rezeptoren die für den Marker bindungsspezifischen zweiten Bindungsstellen aufweist.

Aus DE 100 38 080 A1 ist zwar bereits ein Verfahren bekannt, bei dem an der Oberfläche eines Trägers an einer Teststelle biologische Rezeptoren markiert werden, um Aussagen über die Gesamtzahl möglicher Bindungsplätze für mit einem weiteren Fluoreszenzstoff markierte Antikörper zu erhalten. Aus dem Verhältnis der fixierten Bindungsstellen zur Fluoreszenzstrahlung der gebundenen Antikörper wird dann eine Aussage über die Besetzungsdichte der Bindungsstellen abgeleitet. Bei diesem Verfahren weisen die Rezeptoren jedoch keine zweiten Bindungsstellen auf Deshalb können die Rezeptoren nach dem Messen der Gesamtzahl der möglichen Bindungsplätze nicht mehr zur Messung der Ligandenkonzentration verwendet werden. Für die Messung werden also mindestens zwei gleiche Teststellen bzw. mit Rezeptoren beschichtete Träger benötigt. Die Durchführung des Verfahrens ist also mit einem relativ großen Materialverbrauch und entsprechend hohen Kosten verbunden.

Bei einer bevorzugten Ausgestaltung der Erfindung werden die Rezeptoren in einem Lösungsmittel gelöst derart mit den Markern in Kontakt gebracht, dass diese an die zweiten Bindungsstellen binden und danach werden die so erhaltenen Marker-Rezeptor-Komplexe auf dem Träger immobilisiert. Der Biochip lässt sich dadurch kostengünstig herstellen.

Vorteilhaft ist, wenn nach dem Binden der zweiten Bindungsstellen an die Marker freie Marker aus der das Lösungsmittel, die Marker und die Rezeptoren enthaltenden Lösung entfernt werden, vorzugsweise durch Chromatographie und/oder Dialyse, und wenn die so erhaltene Lösung danach derart mit dem Träger In Kontakt gebracht wird, dass die Marker-Rezeptor-Komplexe auf dem Träger immobilisiert werden. Dadurch wird erreicht, dass ungebundene Marker beim Aufbringen der Rezeptoren auf die Trägeroberfläche nicht mit dieser in Kontakt geraten können. Die Flächenbelegung der Trägeroberfläche mit den Rezeptoren kann dann noch genauer ermittelt werden.

Zweckmäßigerweise wird die Lösung, bestehend zumindest aus dem Lösungsmittel, den Rezeptoren und den Markern mit einer vorgegebenen Menge unmarkierter Marker vermischt, bevor sie mit dem Träger in Kontakt gebracht wird. Dadurch kann die in Abhängigkeit von der Bindung der Marker an die Rezeptoren erzeugte Lumineszenzstrahlung derart abgeschwächt werden, dass ein zur Messung der Lumineszenzstrahlung vorgesehener Detektor in einem günstigen Arbeitspunkt betrieben wird.

Bei einer anderen Ausführungsform der Erfindung werden die Marker an die zweiten Bindungsstellen gebunden nachdem die Rezeptoren auf dem Träger immobilisiert wurden, indem eine die Marker enthaltende Flüssigkeit auf den Träger aufgebracht und nach dem Binden der Marker an die zweiten Bindungsstellen die Flüssigkeit sowie darin eventuelle noch enthaltene freie Marker von dem Träger entfernt werden. Die Rezeptoren können also auch noch nachträglich mit dem Marker markiert werden, nachdem sie auf dem Träger immobilisiert wurden.

Vorteilhaft ist, wenn ein Effektivitätswert für die Anzahl der Marker, die im Mittel an einen auf dem Träger immobilisierten Rezeptor gebunden sind vorzugsweise photometrisch ermittelt wird, und wenn aus dem Messsignal für die Lumineszenzstrahlung und dem Effektivitätswert mindestens ein Messwert für die Flächenbelegung des Trägers mit den Rezeptoren bestimmt wird. Mit Hilfe des Biochips kann dann die Konzentration von In einer zu untersuchenden Probe enthaltenen Liganden quantitativ bestimmt werden, indem die Probe derart mit den Rezeptoren in Kontakt gebracht wird, dass die Liganden an die Rezeptoren binden können, und Indem danach ein Messwert M für die Häufigkeit der Bindungen zwischen den Liganden und den Rezeptoren erfasst und die Konzentration des Liganden unter Berücksichtigung des Messwerts F für die Flächenbelegung nach dem Massenwirkungsgesetz berechnet wird:

$$Ligandenkonzentration = K \, \frac{M}{F}.$$

Dabei bedeutet K eine Bindungskonstante, die beispielsweise experimentell ermittelt werden kann.

Bei einer bevorzugten Ausführungsform wird als Messsignal ein Bildsignal für ein flächiges Abbild mindestens eines die Rezeptoren enthaltenden Oberflächenbereichs des Trägers erfasst Dabei kann das Abbild ein Binärbild (schwarz-weiß Bild) oder ein Grauwertbild sein. Das Bildsignal wird bevorzugt mittels einer Kamera, insbesondere einer CCD-Kamera aufgezeichnet. Es Ist aber auch denkbar, den mindestens einen Oberflächenbereich mit Hilfe eines Scanners abzutasten. Das Abbild der Rezeptoren kann bei der Fertigung des Biochips zur Qualitätsüberprüfung durch eine Überwachungsperson auf einem Monitor angezeigt und/oder mit Methoden der Bildverarbeitung automatisch ausgewertet werden. Es ist aber auch denkbar, das Bildsignal und/oder mindestens eine daraus abgeleitete geometrische Kenngröße für die Oberflächenbelegung des Trägers mit den Rezeptoren, wie zum Beispiel die genaue Lage und/oder die Abmessungen von auf dem Träger befindlichen, mit den Rezeptoren beschichteten Messpunkten und/oder die Flächenbelegungsdichte zu speichern. Dabei ist es sogar möglich, dass in den Träger ein Halbleiterchip integriert ist, der einen nichtflüchligen Datenspeicher enthält, in dem das Bildsignal und/oder die Kenngröße(n) gespeichert werden.

Vorteilhaft ist, wenn das Messsignal mit einem Sollsignal oder einem Sollslgnalbereich verglichen wird und wenn in Abhängigkeit vom Ergebnis dieses Vergleichs die auf dem Träger immobilisierten Rezeptoren mit einem Analyten in Kontakt gebracht werden, von dem vermutet wird, dass er den Ligand enthält. Bei der Serienfertigung von Biochips in einer Fabrik kann dann eine 100%-ige Qualitätsprüfung durchgeführt werden, bei der alle Biochlps, bei denen das Messsignal von dem Sollsignal oder einem Sollsignalbereich abweicht, ausgesondert und nur diejenigen Biochips mit dem Analyten in Kontakt gebracht werden, welche die Qualitätsüberprüfung bestanden haben. Es besteht aber auch die Möglichkeit, einem Biochlp, dessen Messsignal von dem Sollsignal oder dem Sollsignalbereich abweicht, mindestens einen Korrekturwert zuzuordnen, der es ermöglicht, den Biochip dennoch für die Messung der Konzentrafion des Liganden im Analyten zu verwenden, indem eine auf die Abweichung zurückzuführende Änderung des Messsignals mit Hilfe des Korrekturwerts kompensiert wird.

Bei einer bevorzugten Ausgestaltung der Erfindung wird der Marker durch eine Anregungsstrahlung zur Angabe der Lumineszenzstrahlung angeregt. Die Messung der Bindung der Liganden an den Rezeptor erfolgt dann bevorzugt in der Weise, dass in einem Chemilumineszenzsubstrat in Abhängigkeit von der Bindung des Liganden an den Rezeptor eine zweite Lumineszenzstrahlung erzeugt wird. Dabei ist das Chemilumineszenzsubstrat so gewählt, dass an den zur Überprüfung der Flächenbelegung des Trägers mit den Rezeptoren vorgesehenen Markern keine optischer Strahlung erzeugt wird. Die Anregungsstrahlung wird vorteilhaft mit einer von dem Träger beabstandeten externen Lichtquelle erzeugt. Es ist aber auch möglich, dass die Lichtquelle in den Träger Integriert ist, beispielsweise in Form mindestens einer Leuchtdiode.

Bei einer anderen Ausführungsform der Erfindung wird die Lumineszenzstrahlung in Abhängigkeit von der Anwesenheit des Markers mittels einer chemischen Reaktion erzeugt. Die Detektion der Bindung der Liganden an den Rezeptor erfolgt dann bevorzugt in der Weise, dass in Abhänglgkeit von der Bindung des Liganden an den Rezeptor mit Hilfe einer Anregungsstrahlung ein direkt oder indirekt über einen Nachweisantikörper an den Liganden gebundener zweiter Marker zur Abgabe einer zweiten Lumineszenzstrahlung angeregt und die zweite Lumineszenzstrahlung gemessen wird. Dabei ist die Anregungsstrahlung derart auf die zur Überprüfung der Flächenbelegung des Trägers mit den Rezeptoren vorgesehenen Marker abgestimmt, dass diese durch die Anregungsstrahlung nicht zur Aussendung der zweiten Lumineszenzstrahlung angeregt werden.

Vorteilhaft ist, wenn in Abhängigkeit von der Bindung der Liganden an die Rezeptoren eine zweite Lumineszenzstrahlung erzeugt wird, und wenn der Messwert für die Häufigkeit der Bindungen zwischen den Liganden und den Rezeptoren durch Messen der zweiten Lumineszenzstrahlung erfasst wird, vorzugsweise mittels mindestens eines direkt unter den Rezeptoren in den Träger integrierten optischen Sensors. Der optische Sensor ist dann dicht zu den Rezeptoren benachbart, so dass ein entsprechend großes Raumsegment der zweiten Lumineszenzstrahlung auf den optischen Sensor auflrifft. Die zweite Lumineszenzstrahlung kann dann mit hoher Messempfindlichkeit erfasst werden.

Bezüglich des Biochips wird die vorstehend genannte Aufgabe dadurch gelöst, dass die Rezeptoren zusätzlich zu der mindestens einen ersten Bindungsstelle jeweils wenigstens eine zweite Bindungsstelle haben, die für den Liganden nicht bindungsspezifisch ist, und dass an die zweite Bindungsstelle jeweils ein zur Aussendung einer Lumineszenzstrahlung anregbarer optischer Marker gebunden ist.

In vorteilhafter Weise ist es dadurch möglich, mit Hilfe der Marker eine Lumineszenzstrahlung zu erzeugen und diese zur Bestimmung der Flächenbelegung des Trägers mit den Rezeptoren zu messen. Da die Marker über zweite Bindungsstellen an die Rezeptoren gebunden werden, können die ersten Bindungsstellen weiterhin zum Nachweis und/oder zum Bestimmen der Konzentration des Liganden in einer zu untersuchenden Probe genutzt werden. Die Biochips können also individuell überprüft werden.

Weitere vorteilhafte Ausgestaltungen der Erfindung sind in den Unteransprüchen beschrieben.

Nachfolgend ist ein Ausführungsbeispiel der Erfindung anhand der Zeichnung näher erläutert. Es zeigt

Fig. 1    einen Längsschnitt durch einen Biochip, der einen Träger aufweist, auf dem Rezeptoren immobilisiert sind, die mit einem optischen Marker markiert sind,

Fig. 2    den in Fig. 1 gezeigten Biochlp während der Messung der Flächenbelegung des Trägers mit den Rezeptoren,

Fig. 3    eine zweidimensionale graphische Darstellung des Messsignals für die Flächenbelegung des Trägers mit den Rezeptoren,

Fig. 4    den Blochip während des Inkontaktbringens mit einer einen für die Rezeptoren bindungsspezifischen Liganden enthaltenden Probe, und

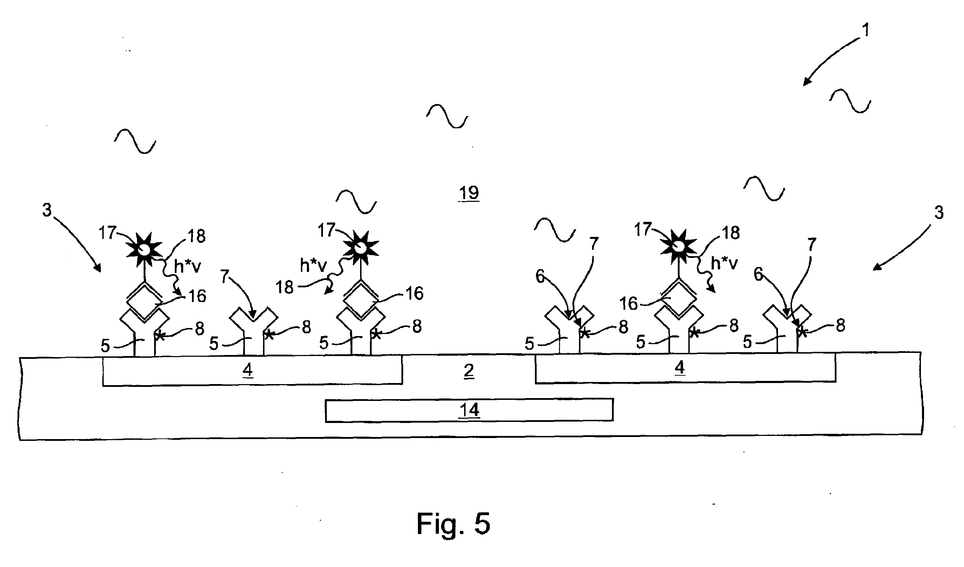

Fig. 5    den Biochip während des Inkontaktbringens mit einem Chemilumineszenzsubstrat.

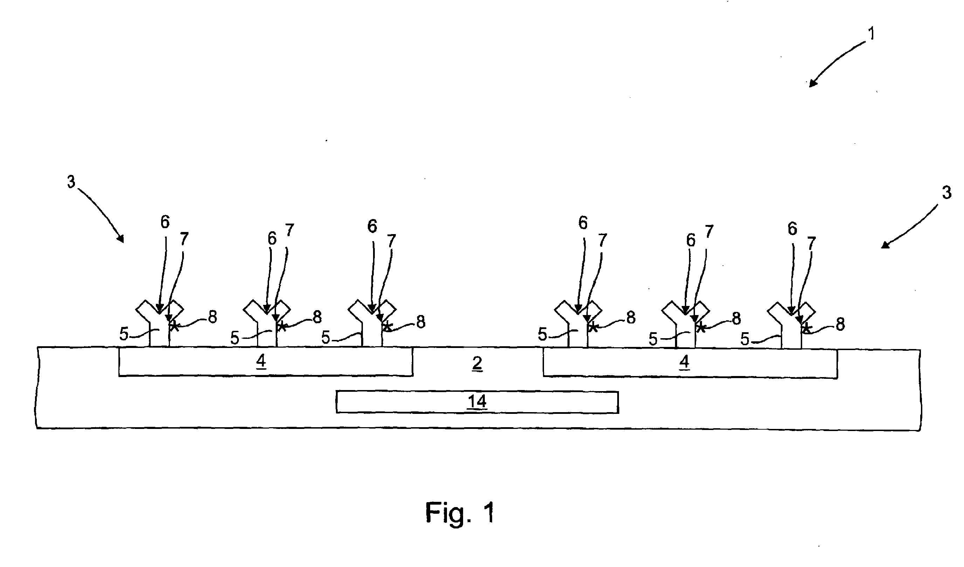

Bei einem Verfahren zur Herstellung eines in Fig. 1 im Ganzen mit 1 bezeichneten Biochips wird ein Träger 2 bereitgestellt, der mehrere Teststellen 3 aufweist, an denen jeweils ein optischer Halbleiter-Sensor 4 in den Träger 2 integriert ist. Die Sensoren 4 sind mit einer in der Zeichnung nicht näher dargestellten Auswerteeinrichtung verbunden, mittels Messsignale aus den Sensoren 4 auslesbar sind.

Außerdem werden Rezeptoren 5 bereitgestellt, die jeweils mindestens eine erste freie Bindungsstelle 6 und wenigstens eine zweite freie Bindungsstelle 7 aufweisen. Die ersten Bindungsstelle 6 ist für einen nachzuweisenden Liganden 9 bindungsspezifisch und die zweite Bindungsstelle 7 ist zum Binden eines optischen Markers 8, nämlich einem NHS-Ester, vorgesehen.

Die Rezeptoren 5 werden in aufgereinigter Form in einem geeigneten Puffer, wie z.B. einem PBS-Puffer mit einem pH-Wert von 7,4 gelöst. Das gewünschte Verhältnis zwischen der molaren Menge des Rezeptors 5 und der molaren Menge des Markers 8 kann durch Wiegen der Rezeptoren 5 und der Marker 8, Herstellen entsprechender Verdünnungen und Mischen der Verdünnungen eingestellt werden. In dem Puffer binden die Marker 8 an die zweiten Bindungsstellen 7, während die ersten Bindungsstellen 6 frei bleiben. Danach werden eventuell noch in der Lösung enthaltene ungebundene Marker 8 durch Chromatographie und/oder Dialyse aus der Lösung entfernt.

In einem weiteren Verfahrensschritt wird ein Wert für die Effizienz der Markierung der Rezeptoren 5 mit den Markern 8 photometrisch bestimmt, indem mindestens ein Messwert für die optische Absorption der Rezeptor-Markerkomplexe gemessen und mit zumindest einem entsprechenden Messwert für unmarkierte Rezeptoren verglichen wird. Aus dem Unterschied zwischen der Absorption der markierten und der unmarkierten Rezeptoren 5 wird ein Effektivitätswert ermittelt.

Nun werden den markierten Rezeptoren 5 im molaren Verhältnis 1:10 unmarkierte Rezeptoren zugemischt. Das so erhaltene Gemisch wird an den Teststellen 3 mit dem Träger 2 in Kontakt gebracht und dann werden die Rezeptoren 5 auf dem Träger 2 immobilisiert, beispielsweise durch Photovernetzung.

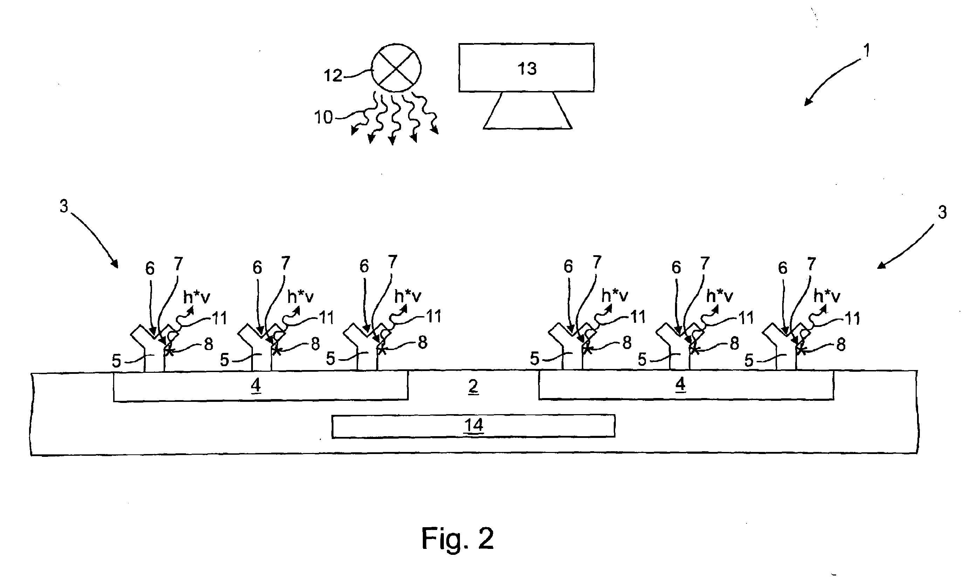

Nachdem der Puffer und eventuell noch vorhandene nicht immobilisierte Rezeptoren 5 und/oder Rezeptor-Marlserkomplexe von der Oberfläche des Trägers 2 entfernt wurden, werden die Teststellen 3 mit einer optischen Anregungsstrahlung 10 bestrahlt (Fig. 2). Die Anregungsstrahlung 10 wird mit Hilfe einer von dem Träger 2 beabstandeten Lichtquelle 12 erzeugt.

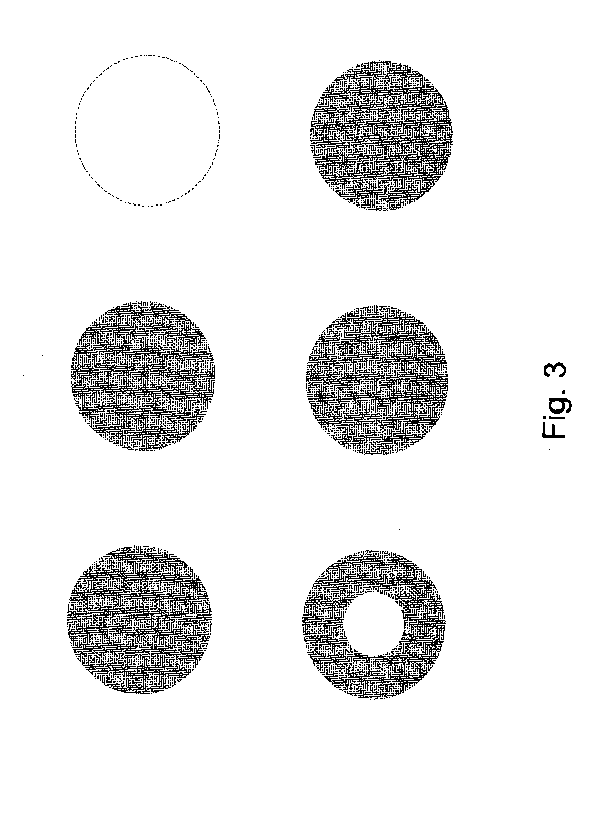

Durch die Anregungsstrahlung 10 werden die Marker 8 zur Aussendung einer ersten Lumineszenzstrahlung 11 angeregt. Die Wellenlänge der ersten Lumineszenzstrahlung 11 unterscheidet sich von der Wellenlänge der Anregungsstrahlung 10. Auf die Teststellen 3 wird ein Detektor 13, wie z.B. eine Kamera, gerichtet, der für die Lumineszenzstrahlung 11 empfindlich und für die Anregungsstrahlung 10 unempfindlich ist. Mittels des Detektors 13 wird ein zweidimensionales Grauwert-Abbild der Oberfläche des Trägers 2 aufgezeichnet (Flg. 3). Es wird also dle Intensität der ersten Lumineszenzstrahlung 11 ortsaufgelöst gemessen.

Mit Hilfe der Messwerte für die Lumineszenzstrahlung 11, der bekannten Lage des Detektors 13 relativ zu dem Biochip 1, KenngröBen für die optischen Abbildungseigenschaften des Detektors 13 und dem zuvor ermittelten Effektivltätswert werden Kenngrößen für die Oberflächenbelegung des Trägers 2 mit den Rezeptoren 5 bestimmt. Die Kenngrößen können ortsaufgelöste Messwerte für die Flächenbelegung des Trägers 2 mit den Rezeptoren 5 sowie die Anordnung der Teststellen 3 auf dem Träger 2 und die individuellen Abmessungen der einzelnen Teststellen 3 umfassen. Die so ermittelten Kenngrößen werden in einem Halbleiterspeicher 14 abgelegt, die in den Träger 2 integriert Ist. Bei Bedarf können in dem Halbleiterspeicher 14 zusätzliche Informationen abgelegt werden, beispielsweise über die Art der Rezeptoren 5. Ferner besteht die Möglichkeit, in dem Halbleiter eine Information über den Zeitpunkt der Messung der Kenngrößen zu speichern. Dadurch ist es möglich, die altersbedingte Abnahme der für den Liganden bindungsspezifischen, aktiven Bindungsstellen bei der Bestimmung der Konzentration des Liganden in einer zu untersuchenden. Probe 15 zu berücksichtigen.

Die ermittelten Kenngrößen werden ferner mit Sollwertbereichen verglichen. Wenn eine Kenngröße außerhalb des ihr zugeordneten Sollwertbereichs liegt, wird der Biochip als defekt ausgesondert und/oder die betreffende Teststelle 3, an der die Abweichung festgestellt wurde, als defekt markiert. In Fig. 3 ist erkennbar, dass beispielsweise die Teststelle 3 links unten nur teilweise und die Teststelle 3 rechts oben überhaupt nicht mit Rezeptoren 5 beschichtet ist.

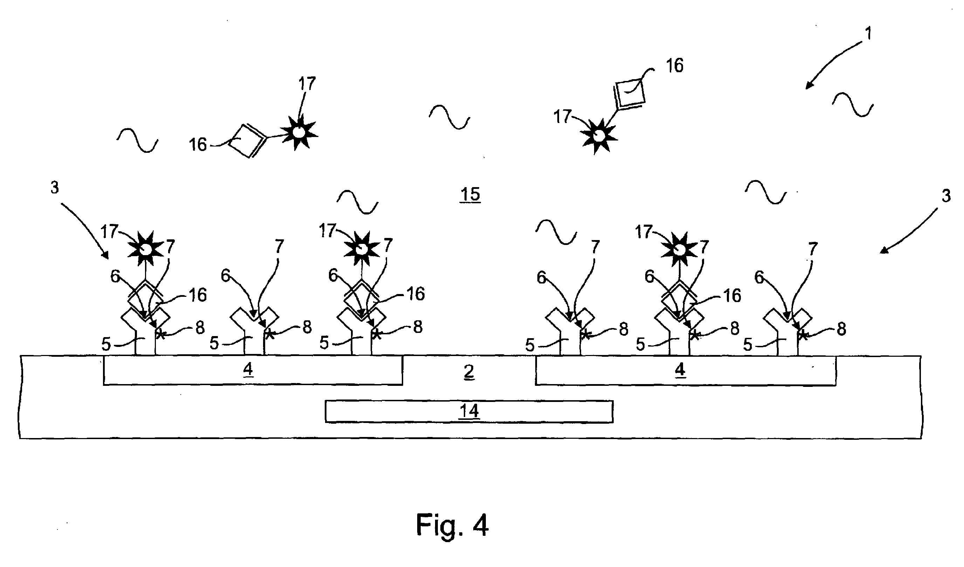

In einem weiteren, in Fig. 4 dargestellten Verfahrensschritt, werden die auf dem Träger 2 immobilislerten Rezeptoren 5 derart mit einer die Liganden 9 enthaltenden Probe 15 in Kontakt gebracht, dass die Liganden 9 an die ersten Bindungsstellen 6 binden. An die Liganden 9 ist jeweils über einen Nachweisantikörper 16 ein zweiter Marker 17, nämlich das Enzym Meerrettich-Peroxidase, gebunden. Eventuell auf der Trägeroberfläche noch vorhandene freie Liganden 9 werden nun von der Trägeroberfläche entfernt, beispielsweise indem der Träger 2 mit einer Spülflüsslgkeit abgespült wird.

Dann werden die Teststellen 3 mit einem Chemilumineszenzsubstrat 19 in Kontakt gebracht, das Wasserstoffperoxid und Luminol enthält. Danach werden Messwerte für die Häufigkeit der Bindungen zwischen den Liganden 9 und den Rezeptoren 5 erfasst, indem in Abhängigkeit von der Anwesenheit des zweiten Markers 17 mittels einer chemischen Reaktion zwischen dem Wasserstoffperoxid und Luminol eine zweite Lumineszenzstrahlung 18 erzeugt und mit Hilfe der Sensoren 4 detektiert wird. Die Messwerte für die Flächenbelegung der Teststellen 3 mit den Rezeptoren und der Zeitpunkt der Erfassung dieser Messwerte werden aus dem Halbleiterspeicher 14 ausgelesen und es wird die Zeitdifferenz zwischen dem zuletzt genannten Zeitpunkt und dem Zeitpunkt der Messung der zweiten Lumineszenzstrahlung bestimmt. Dann wird die Konzentration der Liganden mit Hilfe der Messwerte für die Häufigkeit der Rezeptor-Liganden-Bindungen, der Zeitdifferenz, einem von der Zeitdifferenz abhängigen Korrekturwert und den Messwerten für die Flächenbelegung bestimmt.

**Patentansprüche**

1.  Verfahren zur Herstellung eines Biochips (1), wobei auf einem Träger (2) Rezeptoren (5) immobilisiert werden, die jeweils mindestens eine freie Bindungsstelle (6) haben, die für einen nachzuweisenden Liganden (9) bindungsspezifisch ist, **dadurch gekennzeichnet, dass** zumindest einige Rezeptoren (5) zusätzlich zu der mindestens einen ersten Bindungsstelle (6) jeweils wenigstens eine zweite Bindungsstelle (7) haben, die für den Liganden (9) nicht bindungsspezifisch ist, dass an die zweiten Bindungsstellen (7) optische Marker (8) gebunden werden, dass in Abhängigkeit von der Bindung der einzelnen Marker (8) an die Rezeptoren (5) eine Lumineszenzstrahlung (11) erzeugt wird, und dass zur Überprüfung der Flächenbelegung des Trägers (2)

mit den Rezeptoren (5) ein Messsignal für die Lumineszenzstrahlung (11) erfasst wird.

2. Verfahren nach Anspruch 1, **dadurch gekennzeichnet, dass** die Rezeptoren (5) in einem Lösungsmittel gelöst derart mit den Markern (8) in Kontaktgebracht werden, dass die zweiten Bindungsstellen (7) an die Rezeptoren (5) binden, und dass die so erhaltenen Marker-Rezeptor-Komplexe danach auf dem Träger (2) immobilisiert werden.

3. Verfahren nach Anspruch 1 oder 2, **dadurch gekennzeichnet, dass** nach dem Binden der zweiten Bindungsstellen (7) an die Marker (8) freie Marker (8) aus der das Lösungsmittel, die Marker (8) und die Rezeptoren (5) enthaltenden Lösung entfernt werden, vorzugsweise durch Chromatographie und/ oder Dialyse, und dass die so erhaltene Lösung danach derart mit dem Träger (2) in Kontakt gebracht wird, dass die Marker-Rezeptor-Komplexe auf dem Träger (2) immobilisiert werden.

4. Verfahren nach einem der Ansprüche 1 bis 3, **dadurch gekennzeichnet, dass** die Lösung, bestehend zumindest aus dem Lösungsmittel, den Rezeptoren (5) und den Markern (8) mit einer vorgegebenen Menge unmarkierter Marker (8) vermischt wird, bevor sie mit dem Träger (2) in Kontakt gebracht wird.

5. Verfahren nach einem der Ansprüche 1 bis 4, **dadurch gekennzeichnet, dass** die Marker (8) an die zweiten Bindungsstellen (7) gebunden werden nachdem die Rezeptoren (5) auf dem Träger (2) immobilisiert wurden, indem eine die Marker (8) enthaltende Flüssigkeit auf den Träger aufgebracht und nach dem Binden der Marker (8) an die zweiten Bindungsstellen (7) die Flüssigkeit sowie darin eventuelle noch enthaltene freie Marker (8) von dem Träger (2) entfernt werden.

6. Verfahren nach einem der Ansprüche 1 bis 5, **dadurch gekennzeichnet, dass** ein Effektivitätswert für die Anzahl der Marker (8), die im Mittel an einen auf dem Träger (2) immobilisierten Rezeptor (5) gebunden sind, vorzugsweise photometrisch ermittelt wird, und dass aus dem Messsignal für die Lumineszenzstrahlung und dem Effektivitätswert mindestens ein Messwert für die Flächenbelegung des Trägers (2) mit den Rezeptoren (5) bestimmt wird.

7. Verfahren nach einem der Ansprüche 1 bis 6, **dadurch gekennzeichnet, dass** als Messsignal ein Bildsignal für ein flächiges Abbild mindestens eines die Rezeptoren (5) enthaltenden Oberflächenbereichs des Trägers (2) erfasst wird.

8. Verfahren nach einem der Ansprüche 1 bis 7, **dadurch gekennzeichnet, dass** das Messsignal mit einem Sollsignal oder einem Sollsignalbereich verglichen wird, und dass in Abhängigkeit vom Ergebnis dieses Vergleichs die auf dem Träger (2) immobilisierten Rezeptoren mit einer zu untersuchenden Probe (15) in Kontakt gebracht werden, von der vermutet wird, dass sie den Ligand (9) enthält.

9. Verfahren nach einem der Ansprüche 1 bis 8, **durch gekennzeichnet, dass** der Marker (8) durch eine Anregungsstrahlung zur Angabe der Lumineszenzstrahlung (11) angeregt wird.

10. Verfahren nach einem der Ansprüche 1 bis 9, **dadurch gekennzeichnet, dass** die Lumineszenzstrahlung (11) in Abhängigkeit von der Anwesenheit des Markers (8) mittels einer chemischen Reaktion erzeugt wird.

11. Verfahren zur Bestimmung der Konzentration von in einer zu untersuchenden Probe (15) enthaltenen Liganden (9), wobei die Probe (15) mit den Rezeptoren (5) eines nach dem Verfahren nach einem der Ansprüche 6-10 hergestellten Biochips (1) in Kontakt gebracht wird, wobei mindestens ein die Häufigkeit der Bindungen zwischen den Liganden (9) und den Rezeptoren (5) repräsentierender Messwert erfasst wird, und wobei die Konzentration der Liganden (9) mit Hllfe der Messwerte für die Flächenbelegung und die Häufigkeit der Rezeptor-Liganden-Bindungen bestimmt wird.

12. Verfahren nach Anspruch 11, **dadurch gekennzeichnet, dass** in Abhängigkeit von der Bindung der Liganden (9) an die Rezeptoren (5) eine zweite Lumineszenzstrahlung (18) erzeugt wird, und dass der Messwert für die Häufigkeit der Bindungen zwischen den Liganden (9) und den Rezeptoren (5) durch Messen der zweiten Lumineszenzstrahlung (18) erfasst wird, vorzugsweise mittels mindestens eines direkt unter den Rezeptoren (5) in den Träger (2) Integrierten optischen Sensors (4).

13. Biochip (1), mit einem Träger (2) auf dem Rezeptoren (5) immobilisiert sind, die jeweils mindestens eine freie Blndungsstelle (6) haben, die für einen nachzuweisenden Liganden (9) bindungsspezlfisch ist, **dadurch gekennzeichnet dass** die Rezeptoren (5) zusätzlich zu der mindestens einen ersten Bindungsstelle (6) jeweils wenigstens eine zweite Bindungsstelle (7) haben, die für den Liganden (9) nicht bindungsspezifisch ist, und dass an die zweite Bindungsstelle (9) jeweils ein zur Aussendung einer Lumineszenzstrahlung (11) anregbarer optischer Marker (8) gebunden ist.

14. Blochip (1) nach Anspruch 13, **dadurch gekennzeichnet, dass** die Marker (8) durch eine Anregungsstrahlung (10) zur Aussendung der Lumineszenzstrahlung (11) anregbar sind.

15. Biochip (1) nach Anspruch 13 oder 14, **dadurch gekennzeichnet, dass** die Marker (8) durch eine chemische Reaktion zur Aussendung der Lumineszenzstrahlung (11) anregbar sind.

16. Biochlp (1) nach einem der Ansprüche 13 bis 15, **dadurch gekennzeichnet, dass** direkt unter den Rezeptoren (5) mindestens ein optischer Sensor (4) in den Träger (2) integriert ist, und dass der optische Sensor (4) vorzugsweise für die Lumineszenzstrahlung (11) der Marker (8) unempfindlich ist.

17. Biochip (1) nach einem der Ansprüche 13 bis 16, **dadurch gekennzeichnet, dass** er einen maschinenlesbaren Datenträger, Insbesondere einen Halbleiterspeicher (14) aufweist, in dem mindestens eine Kenngröße für die Oberflächenbelegung des Trägers (2) mit den Rezeptoren (5), insbesondere ein Messwert für die Flächenbelegung des Trägers (2) mit den Rezeptoren (5), und ggf. den Zeitpunkt, an dem die mindestens eine Kenngröße ermittelt wurde, gespeichert ist.

Fig. 1

EP 1 975 247 A1

Fig. 2

EP 1 975 247 A1

Fig. 3

Fig. 4

EP 1 975 247 A1

Fig. 5

Europäisches
Patentamt

**EUROPÄISCHER RECHERCHENBERICHT**

Nummer der Anmeldung

EP 07 00 6650

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | US 6 775 621 B1 (YURINO NORIKO [JP] ET AL)<br>10. August 2004 (2004-08-10)<br>* Spalte 2, Zeilen 23-28 *<br>* Spalte 3, Zeilen 42-44 *<br>* Spalte 4, Zeile 61 - Spalte 7, Zeile 31<br>*<br>* Abbildungen 2-6 *<br>----- | 1,7,9,<br>11-14<br>10,15,16 | INV.<br>C12Q1/68<br>G01N21/64<br>G01N33/543 |
| X<br><br><br><br><br><br><br><br><br><br>Y | HESSNER M J ET AL: "THREE COLOR CDNA MICROARRAYS: QUANTITATIVE ASSESSMENT THROUGH THE USE OF FLUORESCEIN-LABELED PROBES"<br>NUCLEIC ACIDS RESEARCH, OXFORD UNIVERSITY PRESS, SURREY, GB,<br>Bd. 31, Nr. 4,<br>15. Februar 2003 (2003-02-15), Seiten 1-6,<br>XP001182174<br>ISSN: 0305-1048<br>* Zusammenfassung *<br>----- | 1,2,7,9,<br>11-14<br><br><br><br><br><br><br><br>10,15,16 | |
| X<br><br><br>Y | WO 2005/075682 A (PARK HEE-KYUNG [KR]; KIM CHEOL-MIN [KR]; JANG HYUN-JUNG [KR])<br>18. August 2005 (2005-08-18)<br>* Seite 3, Zeile 30 - Seite 4, Zeile 4 *<br>* Seite 5, Zeilen 20-26 *<br>* Seite 7, Zeile 16 - Seite 9, Zeile 20 *<br>* Seite 15, Zeilen 17-25 *<br>* Seite 16, Zeilen 14-18 *<br>* Abbildung 3 *<br>----- | 1,4,7,9,<br>11-14<br><br>10,15,16 | RECHERCHIERTE SACHGEBIETE (IPC)<br><br>G01N<br>C12Q |
| X<br><br><br>Y | WO 2006/001399 A (OLYMPUS CORP [JP];<br>AKIMOTO YOSHINOBU [JP])<br>5. Januar 2006 (2006-01-05)<br>* Seite 11, Zeile 11 - Seite 23, Zeile 14<br>*<br>-----<br>-/-- | 1,2,4,6,<br>8,9,<br>11-14,17<br>10,15,16 | |

Der vorliegende Recherchenbericht wurde für alle Patentansprüche erstellt

| Recherchenort | Abschlußdatum der Recherche | Prüfer |
|---|---|---|
| München | 24. August 2007 | Hoogen, Ricarda |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
.................................................................
& : Mitglied der gleichen Patentfamilie, übereinstimmendes
    Dokument

EPO FORM 1503 03.82 (P04C03)

**EP 1 975 247 A1**

**EUROPÄISCHER RECHERCHENBERICHT**

Nummer der Anmeldung

EP 07 00 6650

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2005/019778 A1 (VOYTA JOHN C [US] ET AL) 27. Januar 2005 (2005-01-27) * Zusammenfassung * ----- | 10,15 | |
| Y | DE 101 45 701 A1 (INFINEON TECHNOLOGIES AG [DE]) 10. April 2003 (2003-04-10) * Zusammenfassung * ----- | 16 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 24. August 2007 | Hoogen, Ricarda |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 07 00 6650

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-08-2007

| Im Recherchenbericht angeführtes Patentdokument | | Datum der Veröffentlichung | Mitglied(er) der Patentfamilie | | Datum der Veröffentlichung |
|---|---|---|---|---|---|
| US 6775621 | B1 | 10-08-2004 | DE<br>EP<br>JP<br>JP | 69930826 T2<br>1010764 A2<br>3442327 B2<br>2000235035 A | 30-11-2006<br>21-06-2000<br>02-09-2003<br>29-08-2000 |
| WO 2005075682 | A | 18-08-2005 | EP<br>US | 1721001 A1<br>2007122816 A1 | 15-11-2006<br>31-05-2007 |
| WO 2006001399 | A | 05-01-2006 | JP | 2006010391 A | 12-01-2006 |
| US 2005019778 | A1 | 27-01-2005 | WO | 2005007874 A2 | 27-01-2005 |
| DE 10145701 | A1 | 10-04-2003 | WO<br>EP<br>JP<br>US | 03027676 A1<br>1428026 A1<br>2005504293 T<br>2004234417 A1 | 03-04-2003<br>16-06-2004<br>10-02-2005<br>25-11-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 10251757 B4
- DE 10038080 A1